## Europäisches Patentamt
## European Patent Office
## Office européen des brevets

(19)

(11) Veröffentlichungsnummer: **0 023 572**
**B1**

(12)

# EUROPÄISCHE PATENTSCHRIFT

(45) Veröffentlichungstag der Patentschrift:
**04.04.84**

(51) Int. Cl.³: **C 07 C 39/16**, C 07 C 37/20,
C 08 G 63/62

(21) Anmeldenummer: **80103721.9**

(22) Anmeldetag: **01.07.80**

(54) **Verfahren zur Herstellung von Gemischen alkylierter aromatischer Polyhydroxyverbindungen, diese Produktmischungen und deren Verwendung zur Herstellung von Polycarbonaten.**

(30) Priorität: **13.07.79 DE 2928443**

(43) Veröffentlichungstag der Anmeldung:
**11.02.81 Patentblatt 81/6**

(45) Bekanntmachung des Hinweises auf die Patenterteilung:
**04.04.84 Patentblatt 84/14**

(84) Benannte Vertragsstaaten:
**DE FR GB IT NL**

(56) Entgegenhaltungen:
**EP - A - 0 001 863
AT - B - 214 436
DE - A - 2 063 050
DE - A - 2 830 174
DE - A - 2 830 175
GB - A - 937 072
GB - A - 1 122 003
GB - A - 1 185 102
GB - A - 1 185 223
US - A - 3 242 219**

(73) Patentinhaber: **BAYER AG, Konzernverwaltung RP
Patentabteilung, D-5090 Leverkusen 1 Bayerwerk (DE)**

(72) Erfinder: **Serini, Volker, Dr., Scheiblerstrasse 81,
D-4150 Krefeld (DE)**
Erfinder: **Neumann, Rainer, Dr.,
Bodelschwinghstrasse 16, D-4150 Krefeld (DE)**
Erfinder: **Friedhofen, Gerhard, Dr., Holzapfelweg 3,
D-4150 Krefeld (DE)**
Erfinder: **Freitag, Dieter, Dr., Hasenheide 10,
D-4150 Krefeld (DE)**
Erfinder: **Heuser, Jürgen, Dr., Doerperhofstrasse 35,
D-4150 Krefeld (DE)**
Erfinder: **Schwarz, Hans-Helmut, Dr., Ratherstrasse 90,
D-4150 Krefeld (DE)**

**Verfahren zur Herstellung von Gemischen alkylierter aromatischer Polyhydroxyverbindungen, diese Produktmischungen und deren Verwendung zur Herstellung von Polycarbonaten**

Gegenstand der vorliegenden Erfindung ist ein Verfahren zur Herstellung von Gemischen alkylierter aromatischer Polyhydroxyverbindungen aus alkylierten Phenolen und gesättigten aliphatischen und cycloaliphatischen $C_1—C_{12}$-Aldehyden und -Ketonen an sauren organischen Ionenaustauschern als Katalysatoren, dadurch gekennzeichnet, daß man als alkyliertes Phenol ein technisches 2,6-Di-$(C_1—C_4$-alkyl)-phenol, bestehend aus 70 bis 98% 2,6-Di-$(C_1—C_4$-alkyl)-phenol, 2 bis 30% Mono-$(C_1—C_4$-alkyl)-phenol, <0,1 bis 5% Phenol und <0,1—2% von mindestens dreifach $(C_1—C_4$-alkyl)-substituiertem Phenol verwendet und aus dem Reaktionsgemisch überschüssiges alkyliertes Phenol und leichtsiedende Substanzen in einem Verdampfer bei Temperaturen zwischen 100°C und 300°C und bei Drücken von 0,05 bis 1000 mbar abtrennt und anschließend bei den gleichen Druck- und Temperaturbedingungen den Rückstand mit Inertgas behandelt zum Entfernen des restlichen alkylierten Phenols bis auf einen Gehalt von weniger als 5 Gew.-%.

Vorzugsweise wird das Verfahren kontinuierlich geführt, wobei das abgetrennte Alkylphenolgemisch ohne Wiederaufarbeitung in die Reaktion zurückgeführt wird.

Gegenstand der Erfindung sind weiterhin die nach dem erfindungsgemäßen Verfahren erhaltenen Gemische alkylierter aromatischer Polyhydroxyverbindungen. Diese können durch Hydrierung zu aliphatischen Polyhydroxyverbindungen umgesetzt werden, die z. B. für die Polyesterherstellung von Bedeutung sind. Sie sind weiterhin durch Hydrierung und Aminierung zu Polyaminen umsetzbar, die eine geringe Kristallisationsneigung aufweisen und für die Synthese von Polyamiden und Polyurethanen sowie für andere Zwecke mit Vorteil einsetzbar sind. Sie können insbesondere auch mit Vorteil zur Herstellung von hochmolekularen Polycarbonaten mit ausgezeichneten Eigenschaften eingesetzt werden. Gegenstand der Erfindung sind deshalb auch die Verwendung der erfindungsgemäß erhaltenen Gemische alkylierter aromatischer Polyhydroxyverbindungen zur Herstellung hochmolekularer, thermoplastischer Polycarbonate. Die Herstellung der hochmolekularen Polycarbonate kann nach an sich bekannten Methoden erfolgen (siehe zum Beispiel DE-OS 2 063 050, 2 063 052, 1 570 703, 2 211 956 und 2 211 957).

2,6-Dimethylphenol wird technisch überwiegend durch Alkylierung von Phenol mit Methanol an anorganischen Katalysatoren erhalten (Ullmann, Encyclopädie der technischen Chemie, Ergänzungsband (1970) 185). Bei der Alkylierung fallen im allgemeinen je nach den Reaktionsbedingungen neben restlichem, nicht umgesetztem Phenol 2,6-Dimethylphenol, mehr oder weniger o-Kresol, p-Kresol, m-Kresol und weitere, mindestens zweifach alkylierte Phenole an. Das 2,6-Dimethylphenol wird im allgemeinen durch Destillation aus dem Alkylat abgetrennt, wenn der Anteil der anderen alkylierten Phenole und des Phenols für den Verwendungszweck des 2,6-Dimethylphenols zu hoch ist. Sehr reines 2,6-Dimethylphenol, wie es bisher für die Herstellung von Poly-(2,6-dimethylphenyl)-oxid oder 2,2-Bis-(3,5-dimethyl-4-hydroxyphenyl)-propan benötigt wird, ist nur durch erhöhten Destillationsaufwand oder mit Hilfe anderer aufwendiger Reinigungsoperationen, wie z. B. Kristallisation, erhältlich.

Bei bekannten technischen Verfahren der Alkylierung von Phenolen mit Methanol (Chem. Processing 1966, November, S. 113 und Chem. Processing 1968, April, S. 32—35) fallen beispielsweise 85—99%ige 2,6-Dimethylphenole an, die für die Herstellung von Polymeren noch weiter gereinigt werden müssen.

Die Kosten des 2,6-Dimethylphenols steigen beträchtlich mit dem Grad der Reinheit, da die obengenannten Reinigungsoperationen technisch aufwendig sind. So ist beispielsweise der Preis eines 99,5%igen 2,6-Dimethylphenols etwa fünfmal so hoch wie der eines etwa 90%igen Materials (Chem. Processing 1966, November, S. 133). Die Verunreinigungen in den technischen 2,6-Dimethylphenolen sind im allgemeinen überwiegend o-, m- und p-Kresol (von 2 bis 30%) neben untergeordneten Mengen Phenol (von <0,1 bis 5%) und mehrfach alkylierten Phenolen (von <0,1 bis 2%).

Annähernd dieselben Verhältnisse liegen auch bei den übrigen bekannten technisch dialkylierten Phenolen vor.

Es ist bekannt, daß aus reinen Phenolen, wie z. B. Phenol, o-Kresol oder 2,6-Dimethylphenol und Carbonylverbindungen mit Hilfe saurer Katalysatoren und ggf. S-haltiger Cokatalysatoren aromatische Dihydroxyverbindungen erhältlich sind (H. Schnell, Chemistry and Physics of Polycarbonates, New York—London—Sydney, Interscience Publishers 1964, Polymer Reviews, Vol. 9 und deutsche Offenlegungsschrift 2 537 027).

Als saure Katalysatoren können z. B. HCl oder saure organische Ionentauscher (US-PS 3 394 089, 3 049 568, DE-OS 2 537 027, 2 830 174 und 2 830 175, GB-PS 937 072 und EP-OS 1 863) dienen. Ionenaustauscher sind z. B. sulfonierte Phenol-Formaldehydharze oder sulfonierte, mit 2—25 Gew.-%, vorzugsweise 6—20 Gew.-% Divinylbenzol vernetzte Polystyrole.

Überraschenderweise wurde nun gefunden, daß es auch gelingt, die vorstehend genannten, technischen 2,6-Dialkylphenole mit Gehalten von 70—98%, bevorzugt von 80—98%, besonders bevorzugt von 85—95% an 2,6-Dialkylphenol, vorzugsweise 2,6-Dimethylphenol, mit dem vorstehend genannten Carbonylverbindungen an sauren, organischen Ionentauschern unter den vorstehend genannten Bedingungen vollständig zu alkylierten aromatischen Polyhydroxyverbindungen umzusetzen, die zur Herstellung von Polycarbonaten geeignet sind.

Wider Erwarten reagieren bei Verwendung saurer organischer Ionentauscher die in den technischen 2,6-Di-($C_1 - C_4$-alkyl)-phenolen, vorzugsweise 2,6-Dimethylphenolen, unterschiedlich alkylierten Phenole annähernd gleich schnell zu aromatischen Polyhydroxyverbindungen ab.

Überraschend ist weiterhin, daß nach Abdestillation überschüssiger Phenole sowie leichtsiedender Substanzen und nach Desorption restlicher Monophenole, wie im folgenden näher beschrieben, sogar der gesamte Rückstand ohne weitere Reinigung zu hochmolekularen hochwertigen Polycarbonaten umsetzbar ist.

Das erfindungsgemäße Verfahren hat den Vorteil, daß bei nicht vollständigem Umsatz oder bei Verfahren, bei denen die Ausgangsphenole im Überschuß eingesetzt werden (US-PS 3 394 089, 3 049 568, DE-OS 2 537 027), die Zusammensetzung der abgetrennten, nicht umgesetzten Ausgangsphenole gleich bleibt. Dies macht ihren Wiedereinsatz unter Bildung immer gleichartiger Endprodukte möglich. Es braucht also bei der Synthese kein Ausschleusen eines Teils der nicht umgesetzten Ausgangsphenole und ihr Ersatz durch frisches Ausgangsphenol stattfinden.

Damit entfällt auch die Wiederaufarbeitung des ausgeschleusten Materials. Der Umsatz des Ausgangsphenolgemisches ist somit vollständig, so daß die Ausbeute 100% beträgt. Die außerordentlich hohe Wirtschaftlichkeit des Verfahrens beruht auf der Möglichkeit, technische 2,6-Di-($C_1 - C_4$-alkyl)-phenole geringen Reinheitsgrades vollständig zu Gemischen von alkylierten aromatischen Polyhydroxyverbindungen, vorzugsweise alkylierten aromatischen Dihydroxyverbindungen, umsetzen zu können, die noch dazu auf sehr einfache Weise ohne Verluste aufgearbeitet werden können, wie im folgenden beschrieben.

Andere Säuren als die sauren organischen Ionentauscher führen nicht zu dem überraschenden Ergebnis. Mit HCl beispielsweise werden die Kresole stark abgereichert, so daß keine gleichbleibende Zusammensetzung des Rückphenols gewährleistet ist. Mit Schwefelsäure werden zum Beispiel große Mengen sulfonierter Nebenprodukte gebildet, die für die Polycarbonatsynthese ungeeignet sind, so daß große Materialverluste entstehen und eine einfache Aufarbeitung, wie beschrieben, nicht möglich ist.

Als technische 2,6-Dialkylphenole werden solche mit jeweils 1 – 4 C-Atomen in den Alkylgruppen, besonders bevorzugt technisches 2,6-Dimethylphenol, verwendet. Die entsprechenden technischen 2,6-Dialkylphenole bestehen im allgemeinen

aus 98 – 70% 2,6-Dialkylphenol,
aus 2 – 30% Monoalkylphenol,
aus <0,1 – 5% Phenol und
aus <0,1 – 2% von mindestens 3fach alkylierten Phenolen.

Als Carbonylkomponente können alle gesättigten aliphatischen und cycloaliphatischen Aldehyde und Ketone mit $C_1 - C_{12}$, vorzugsweise $C_1 - C_6$ verwendet werden.

Besonders geeignete Carbonylverbindungen sind aufgrund ihrer guten Verfügbarkeit Formaldehyd, Acetaldehyd, Propionaldehyd, Aceton, Butanon und Cyclohexanon.

Die erfindungsgemäße Reaktion wird normalerweise bei Atmosphärendruck durchgeführt. In manchen Fällen kann jedoch Überdruck zweckmäßig sein, um eine optimale Reaktionstemperatur zu erreichen. Die Reaktion läuft im allgemeinen unter 120° C, bevorzugt zwischen 50 und 80° C ab.

Das Molverhältnis Dialkylphenol-Gemisch : Aldehyd bzw. Keton beträgt mindestens 2 : 1. Eine obere Grenze des Molverhältnisses ist aus chemischen Gründen nicht gegeben. Sie hängt ab von der Reaktivität der Reaktionspartner, der Reaktionstemperatur und der Reaktionszeit. Normalerweise wird jedoch ein Verhältnis von 40 : 1 nicht überschritten.

Die Reaktion kann in Gegenwart von Lösungsmitteln durchgeführt werden. Vorzugsweise werden unpolare Lösungsmittel, z. B. Kohlenwasserstoffe wie Toluol, Benzol oder Hexan eingesetzt. Durch diesen Lösungsmittelzusatz kann ein Auskristallisieren der aromatischen Polyhydroxyverbindungen auch bei geringem Überschuß an Dialkylphenol vermieden werden.

Das Verfahren kann diskontinuierlich oder kontinuierlich durchgeführt werden. Bei kontinuierlicher Verfahrensweise kann die Reaktion in einem Wirbel- oder Festbettreaktor, vorzugsweise letzterem, durchgeführt werden. Die kontinuierliche Reaktion ist bevorzugt.

Die Isolierung der gewünschen alkylierten aromatischen Polyhydroxyverbindungen aus dem Reaktionsgemisch erfolgt, indem man aus dem Reaktionsgemisch überschüssiges Phenol und leichtsiedende Substanzen in einem Verdampfer bei Temperaturen zwischen 100° C und 300° C, vorzugsweise bei Temperaturen zwischen 150° C und 250° C, und bei Drücken zwischen 0,05 und 1000 Millibar, vorzugsweise zwischen 0,5 und 500 Millibar abtrennt und anschließend mittels Desorption im gleichen Temperaturbereich und im gleichen Druckbereich unter Verwendung von Inertgas, insbesondere von Stickstoff, die restlichen Monophenole bis auf einen Gehalt von weniger als 5 Gew.-%, vorzugsweise von weniger als 0,5 Gew.-% an Monophenolen, bezogen auf Rückstand, entfernt.

Die Gemische alkylierter aromatischer Polyhydroxyverbindungen, vorzugsweise Alkylbisphenolgemische, können als Sumpfprodukte direkt zur Polycarbonatherstellung eingesetzt werden und die

3

abgetrennten Monophenole wieder in das erfindungsgemäße Verfahren zurückgeschleust werden.

Es zeigte sich, daß die Verwendung der erfindungsgemäßen Gemische alkylierter aromatischer Polyhydroxyverbindungen für die Polycarbonatherstellung besondere Vorteile mit sich bringt. So ist die Löslichkeit der Alkalisalze der erfindungsgemäßen Gemische der Polyhydroxyverbindungen in Wasser besonders gut, was für das Zweiphasengrenzflächenverfahren zur Polycarbonatherstellung von Vorteil ist. Auch ist die Kristallisationsneigung der Polycarbonate aus den erfindungsgemäßen Gemischen von Polyhydroxyverbindungen besonders gering, was sowohl bei der Polycarbonatherstellung als auch bei der Verwendung der Polycarbonate von Bedeutung ist.

Die erfindungsgemäß erhaltenen hochmolekularen Polycarbonate weisen mittlere Molekulargewichte $\overline{M}_w$ von mindestens 20 000, bevorzugt von mindestens 25 000 auf. Es sind mittlere Molekulargewichte bis über $\overline{M}_w = 200\,000$ erreichbar. Im allgemeinen werden für die Anwendung auf dem Thermoplastensektor jedoch mittlere Molekulargewichte unter $\overline{M}_w = 100\,000$, insbesondere unter 60 000 bevorzugt.

Die erfindungsgemäß erhaltenen Polycarbonate aus Gemischen alkylierter aromatischer Polyhydroxyverbindungen weisen überraschenderweise weitgehend die gleichen guten Eigenschaften auf, wie die herkömmlichen Polycarbonate aus reinen alkylierten aromatischen Dihydroxyverbindungen. Sie zeigen zusätzlich bei einer Reihe von Eigenschaften sogar beträchtliche Verbesserungen. So besitzen sie im allgemeinen eine außerordentlich gute Löslichkeit in organischen Lösungsmitteln, insbesondere auch in Lacklösemitteln und in polymerisierbaren Monomeren, wie z. B. Styrol, Acrylnitril, Vinylchlorid, Methylmethacrylat und Mischungen davon, was für Pfropfreaktionen von Vorteil ist. Sie können somit als Basis für die Herstellung von Pfropfpolymeren dienen. Sie zeigen außerdem verbesserte rheologische Eigenschaften in der Schmelze, so höhere Fließfähigkeit und verbesserte Strukturviskosität. Verbessert wird weiterhin die Vernetzbarkeit von Filmen, z. B. durch Elektronenstrahlen, die für verschiedene Anwendungen von Bedeutung ist. Auch ist die Flammfestmachung in vielen Fällen dadurch erleichtert, daß für die gleiche Flammwidrigkeit geringere Mengen an Flammfestmitteln (wie z. B. cokondensiertes 2,2-Bis-(3,5-dibrom-4-hydroxyphenyl)-propan benötigt werden. In vielen Fällen wird auch die Kriechstromfestigkeit deutlich erhöht.

Die erfindungsgemäß erhaltenen Polycarbonate aus Gemischen alkylierter aromatischer Polyhydroxyverbindungen zeigen Verträglichkeiten mit anderen Polymeren, wie sie von Polycarbonaten aus reinen alkylierten aromatischen Polyhydroxyverbindungen her bekannt sind. Dies ist besonders überraschend, da bekannt ist, daß schon kleine Veränderungen in Polymeren oft bedeutende Veränderungen der Verträglichkeit und somit der Eigenschaften mit sich bringen. So ist beispielsweise die Verträglichkeit von Polycarbonaten auf Basis von Gemischen methylierter aromatischer Polyhydroxyverbindungen mit überwiegend 2,2-Bis-(3,5-dimethyl-4-hydroxyphenyl)-propan gegenüber Polycarbonaten auf Basis des entsprechenden reinen Bisphenols mit Polyvinylchlorid (vgl. DE-OS 2 402 176), mit Polycarbonaten auf Basis von 2,2-Bis-(4-hydroxyphenyl)-propan (vergl. DE-OS 2 248 818) und mit Styrolpolymerisaten (vergl. DE-OS 2 329 585 und DE-OS 2 329 646) praktisch nicht verändert. Infolgedessen werden auch hervorragende Polymerlegierungen mit den erfindungsgemäßen Polycarbonaten erhalten.

Von Bedeutung ist ebenso der Erhalt der Hydrolysestabilität der erfindungsgemäß erhaltenen Polycarbonate, insbesondere der von Polycarbonaten auf Basis von Gemischen methylierter aromatischer Polyhydroxyverbindungen mit überwiegend 2,2-Bis-(3,5-dimethyl-4-hydroxyphenyl)-propan (vgl. DE-OS 2 063 050, DE-OS 2 211 957).

Besonders überraschend sind die oben beschriebenen Eigenschaften der erfindungsgemäß erhaltenen Polycarbonate, die aus Gemischen alkylierter aromatischer Polyhydroxyverbindungen hergestellt werden können, die als Sumpfprodukte (siehe oben) isoliert werden. Bei diesen Polycarbonaten sind die teilweise geschilderten guten Eigenschaften der Bisphenole und der geschilderten zusätzlichen Verbesserungen der Polycarbonate noch stärker ausgeprägt.

Die erfindungsgemäß erhaltenen Polycarbonate lassen sich sehr gut zu Formkörpern, Überzügen, Fasern, Lacken und Folien verarbeiten. Mit Vorteil können auch bestimmte Legierungen mit anderen Polymeren, wie z. B. Polyvinylchlorid, Styrolpolymerisate und Polycarbonate aus gereinigten Bisphenolen eingesetzt werden. Die erfindungsgemäß erhaltenen Polycarbonate und ihre Polymerlegierungen lassen sich auch in Mischungen mit Füllstoffen, wie z. B. Mineralien, Holzmehl und Ruß, Verstärkungsstoffen, wie z. B. Glasfasern, Asbest und Kohlefasern, Effektstoffen, Farbstoffen, Pigmenten, Stabilisatoren, z. B. zur Thermo-, Oxidations- und UV-Stabilisierung, Gleit- und Entformungsmitteln, flammfestmachenden Zusätzen, wie z. B. halogenierten aromatischen Verbindungen, Metalloxiden und Metallsalzen, und weiteren Zusatzstoffen gut verwenden. Sie sind insbesondere dort von Vorteil, wo gute elektrische Eigenschaften, hohe Wärmestandfestigkeit, hohe Zähigkeit und hohe Verseifungsstabilität gefordert werden. So können sie beispielsweise für hochwertige elektrische Bauteile, Elektroisolierfolien, Rohrleitungen für alkalische und saure Wasser, Gehäuse, Teile für den Automobilsektor und Haushaltsgeräte dienen.

### Beispiel

Ein 92,8 Gew.-% 2,6-Dimethylphenol, 7,2 Gew.-% o-, m-, p-Kresol und <0,1% Phenol enthaltendes technisches 2,6-Dimethylphenol wird unter Zugabe von Aceton (im Molverhältnis 1 : 15, bezogen auf 2,6-Dimethylphenol) und 1‰ $\beta$-Mercaptopropionsäure durch einen mit trockenem, saurem Ionenaustauscher (sulfoniertes Harz, Perlpolymerisat Styrol/Divinylbenzol 82/18 Gew.-%) gefüllten Festbettreaktor bei einer Temperatur von 70°C und einer Verweilzeit von ca. 20 Stunden gefördert.

Die ablaufende Reaktionslösung enthält, Aceton, Wasser und $\beta$-Mercaptopropionsäure nicht mitgerechnet, 6,5 Gew.-% Kresole, <0,1% Phenol, 84,5 Gew.-% 2,6-Dimethylphenol, 8,1 Gew.-% 2,2-Bis-(3,5-dimethyl-4-hydroxyphenyl)-propan und mindestens 5 weitere, mindestens zweikernige Komponenten (Kondensationsprodukte) zu insgesamt 0,9% (gaschromatographische Analyse nach Silylierung).

Die Reaktionslösung wird in einem ölbeheizten Schlangenverdampfer mit 3,5 m Rohrlänge und 10 mm Rohrdurchmesser bei einer Temperatur von 190°C bei 27 mbar kontinuierlich durchgesetzt. 2,6-Dimethylphenol wird über einen Zyklon, der mit einer kurzen Füllkörperkolonne versehen ist, und in einem Kühler, der bei 50°C betrieben wird, kondensiert. Das 2,2-Bis-(3,5-dimethyl-4-hydroxyphenyl)-propan, das noch 1,5% 2,6-Dimethylphenol enthält, strömt aus dem Schlangenverdampfer in einen Dünnschichtverdampfer mit Rotor als Desorber, der mit Öl auf 185°C geheizt ist und unter dem gleichen Druck wie der Schlangenverdampfer betrieben wird. In den Desorber wird von unten ein Stickstoffstrom von 6 l/h eingeleitet.

Das Rohbisphenol (180 g/h) wird in einer Vorlage aufgefangen. Es enthält unter 0,1% Phenol, 2,6-Dimethylphenol und Kresole, 10,2% unbekannte Komponenten und 84,5% Bis-(3,5-dimethyl-4-hydroxyphenyl)-propan (Rohtetramethylbisphenol A).

Das abgetrennte 2,6-Dimethylphenol wird ergänzt um die zum Tetramethylbisphenol A verlorengegangene Menge und mit dem notwendigen Acetonanteil erneut zur Reaktion gebracht und anschließend wie oben aufgearbeitet.

Selbst nach 10 Wiederholungen ist weder im 2,6-Dimethylphenol noch im Diphenylalkangemisch eine wesentliche Abweichung zum ersten Versuch festzustellen.

In 2286 ml destilliertem Wasser und 614,4 ml 45%iger Natronlauge werden 568,8 g des oben gewonnenen Rohtetramethylbisphenol A und 5,6 g Phenol gelöst. Nach Zugabe von 1490 ml Methylenchlorid, 448 ml Chlorbenzol, 7,72 g Tetrabutylammoniumbromid und 5,70 ml Tri-n-butylamin werden unter Rühren 336,4 g Phosgen gasförmig in 60 min in das Gemisch geleitet. Nach der Einleitung von 250 g Phosgen werden noch 96 ml 45%iger Natronlauge zugesetzt. Nach Zugabe von 5,6 ml Triäthylamin wird 30 min gerührt. Die organische Phase wird dann zur Aufarbeitung nach dem Ansäuern mit verdünnter $H_3PO_2$ und Verdünnen mit Methylenchlorid mit $H_2O$ elektrolytfrei gewaschen. Das durch Abdampfen des organischen Lösungsmittels erhältliche Polycarbonat hat eine relative Viskosität $\eta$rel = 1,296 (gemessen an 0,5 g Polycarbonat in 100 ml $CH_2Cl_2$-Lösung bei 25°C) und zeigt hervorragende Eigenschaften.

### Vergleichsbeispiel

610 Gew.-Teile eines 91,1 Gew.-% Dimethylphenol, 8,9 Gew.-% o-, m-, p-Kresol und <0,1 Gew.-% Phenol enthaltenden technischen Dimethylphenoles wurden unter Zugabe von 19,3 Gew.-Teilen Aceton (im Molverhältnis 1 : 15, bezogen auf technisches 2,6-Dimethylphenol), 1‰ $\beta$-Mercaptopropionsäure und 10 Gew.-Teilen konzentrierter HCl in einem mit Thermometer, Rührer und Rückflußkühler versehenen 3-Halskolben 20 h lang bei 70°C zur Reaktion gebracht.

Die Reaktionslösung enthielt: 6,9 Gew.-% Kresole, <0,1 Gew.-% Phenol, 87,8 Gew.-% 2,6-Dimethylphenol, 4,5 Gew.-% 2,2-Bis-(3,5-dimethyl-4-hydroxy-phenyl)-propan und mindestens 5 weitere, mindestens zweikernige Komponenten (Kondensationsprodukte) zu insgesamt 0,7 Gew.-% (gaschromatographische Analyse nach Silylierung), Aceton, Wasser und $\beta$-Mercaptopropionsäure, wie in Beispiel 1, nicht mitgerechnet.

Schon nach einmaligem Einsatz des technischen 2,6-Dimethylphenols ist in diesem Beispiel die Zusammensetzung des restlichen, nicht umgesetzten 2,6-Dimethylphenols durch Abreicherung der Kresole um 1,5 Gew.-% und Anreicherung des 2,6-Dimethylphenols um den gleichen Wert so verändert, daß eine Wiederverwendung der nicht umgesetzten Ausgangsverbindung mit der Zielsetzung, bei jedem weiteren Einsatz gleichartige Ware zu erhalten, nicht gewährleistet ist.

## Patentansprüche

1. Verfahren zur Herstellung von Gemischen alkylierter aromatischer Polyhydroxyverbindungen, die zur Herstellung hochmolekularer Polycarbonate geeignet sind, durch Umsetzung von alkylierten Phenolen mit gesättigten aliphatischen und cycloaliphatischen $C_1 - C_{12}$-Aldehyden und -Ketonen an sauren organischen Ionenaustauschern als Katalysatoren, dadurch gekennzeichnet, daß man als alkyliertes Phenol ein technisches 2,6-Di-($C_1$—$C_4$-alkyl)-phenol, bestehend aus 70 bis 98% 2,6-Di-($C_1$—$C_4$-alkyl)-phenol, 2 bis 30% Mono-($C_1$—$C_4$-alkyl)-phenol, <0,1 bis 5% Phenol und <0,1—2% von mindestens dreifach ($C_1$—$C_4$-alkyl)-substituiertem Phenol verwendet und aus dem Reaktionsgemisch überschüssiges alkyliertes Phenol und leichtsiedende Substanzen in einem Verdampfer bei Temperaturen zwischen 100°C und 300°C und bei Drücken von 0,05 bis 1000 mbar abtrennt und anschließend bei den gleichen Druck- und Temperaturbedingungen den Rückstand mit Inertgas behandelt zum Entfernen des restlichen alkylierten Phenols bis auf einen Gehalt von weniger als 5 Gew.-%.

2. Verfahren nach Anspruch 1, dadurch gekennzeichnet, daß das Verfahren kontinuierlich geführt wird, wobei das abgetrennte Alkylphenolgemisch ohne Wiederaufarbeitung in die Reaktion zurückgeführt wird.

3. Gemische alkylierter aromatischer Polyhydroxyverbindungen, erhalten nach Anspruch 1.

4. Verwendung der Gemische alkylierter aromatischer Polyhydroxyverbindungen nach Anspruch 3 zur Herstellung von hochmolekularen thermoplastischen Polycarbonaten.

## Claims

1. Process for the production of mixtures of alkylated aromatic polyhydroxy compounds which are suitable for the production of high molecular weight polycarbonates by reacting alkylated phenols with saturated aliphatic and cycloaliphatic $C_1$—$C_{12}$-aldehydes and $C_1$—$C_{12}$-ketones in the presence of acid organic ion exchangers as catalysts, characterised in that the alkylated phenol used is a commercial 2,6-di-($C_1$—$C_4$-alkyl)-phenol consisting of 70 to 98% of 2,6-di-($C_1$—$C_4$-alkyl)-phenol, 2 to 30% of mono-($C_1$—$C_4$-alkyl)-phenol, <0.1 to 5% of phenol and <0.1—2% of phenol which is substituted at least three times by $C_1$—$C_4$-alkyl and excess alkylated phenol and low-boiling substances are separated off from the reaction mixture in an evaporator at temperatures between 100°C and 300°C and under pressures of 0.05 to 1000 mbar and then the residue is treated with inert gas under the same pressure and temperature conditions in order to remove the residual alkylated phenol until the content thereof is less than 5% by weight.

2. Process according to Claim 1, characterised in that the process is carried out continuously, the alkyl phenol mixture separated off being recycled into the reaction without being worked up again.

3. Mixtures of alkylated aromatic polyhydroxy compounds obtained according to Claim 1.

4. Use of the mixtures of alkylated aromatic polyhydroxy compounds according to Claim 3 for the production of high molecular weight thermoplastic polycarbonates.

## Revendications

1. Procédé de production de mélanges de composés polyhydroxyliques aromatiques alkylés qui conviennent à l'obtention de polycarbonates de haut poids moléculaire, par réaction de phénols alkylés avec des aldéhydes et des cétones en $C_1$ à $C_{12}$ aliphatiques et cycloaliphatiques saturés, sur des échangeurs ioniques organiques acides utilisés comme catalyseurs, caractérisé en ce qu'on utilise comme phénol alkylé un 2,6-di-(alkyle en $C_1$—$C_4$)-phénol technique formé de 70 à 98% de 2,6-di-(alkyle en $C_1$—$C_4$)-phénol, de 2 à 30% de mono-(alkyle en $C_1$ à $C_4$)-phénol, de moins de 0,1 à 5% de phénol et de moins de 0,1 à 2% de phénol portant au moins trois substituants alkyle en $C_1$ à $C_4$, et on sépare du mélange réactionnel le phénol alkylé en excès et les substances de bas point d'ébullition dans un évaporateur à des températures comprises entre 100 et 300°C et à des pressions de 0,05 à 1000 mbars, puis on traite le résidu avec un gaz inerte dans les mêmes conditions de pression et de température pour éliminer le phénol alkylé résiduel jusqu'à une teneur de moins de 5% en poids.

2. Procédé suivant la revendication 1, caractérisé en ce qu'il est mis en œuvre en continu, le mélange séparé d'alkylphénols étant recyclé dans la réaction sans nouveau traitement.

3. Mélanges de composés polyhydroxyliques aromatiques alkylés obtenus suivant la revendication 1.

4. Utilisation des mélanges de composés polyhydroxyliques aromatiques alkylés suivant la revendication 3 pour l'obtention de polycarbonates thermoplastiques de haut poids moléculaire.